(19)

**Europäisches Patentamt**
**European Patent Office**
**Office européen des brevets**

(11) **EP 3 892 858 A2**

(12) **EUROPEAN PATENT APPLICATION**

(43) Date of publication:
**13.10.2021 Bulletin 2021/41**

(51) Int Cl.:
**F04B 49/20** *(2006.01)*

(21) Application number: **21167221.7**

(22) Date of filing: **07.04.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.04.2020 JP 2020070046**

(71) Applicant: **Sumitomo Rubber Industries, Ltd.**
**Kobe-shi, Hyogo-ken 651-0072 (JP)**

(72) Inventor: **NAGAHAMA, Masamune**
**Kobe-shi, Hyogo-ken 651-0072 (JP)**

(74) Representative: **Manitz Finsterwald**
**Patent- und Rechtsanwaltspartnerschaft mbB**
**Martin-Greif-Strasse 1**
**80336 München (DE)**

(54) **PUMP SYSTEM**

(57)    A pump system including: a pump configured to intermittently perform an ejecting operation that ejects a fluid; a flow meter configured to measure a flow rate of the fluid ejected from the pump; and a control unit connected to the pump and the flow meter, configured to decide on a length of a stopping section during which the ejecting operation is stopped such that an ejection amount of fluid excessively ejected from the pump is canceled, based on a measured flow rate, which is the flow rate measured by the flow meter, and a target flow rate of the pump set in advance, and configured to control the pump so as to stop the ejecting operation according to the length of the stopping section.

EP 3 892 858 A2

**Description**

The disclosure relates to a pump system.

JP 2011-160868A (hereinafter referred to as "Patent Literature 1") discloses a pump using a MEMS (micro electro mechanical systems) technique. According to Patent Literature 1, the flow rate of a fluid sent by the pump is measured by a flow rate sensor, on/off control of the pump is performed through feedback control that performs feedback of the flow rate measured by the flow rate sensor, and thus the pump is controlled such that a target flow rate is achieved.

When attempting to achieve a target flow rate of a pump through an intermittent operation of the pump as in Patent Literature 1, control is often performed in which the ejection amount of fluid ejected by a current point in time and the ejection amount of fluid that is to be ejected by the current point in time are compared, and, if the former is smaller than the latter, the pump is turned on, or otherwise the pump is turned off. The former ejection amount can be calculated from the measured flow rate, and the latter ejection amount can be set from the target flow rate.

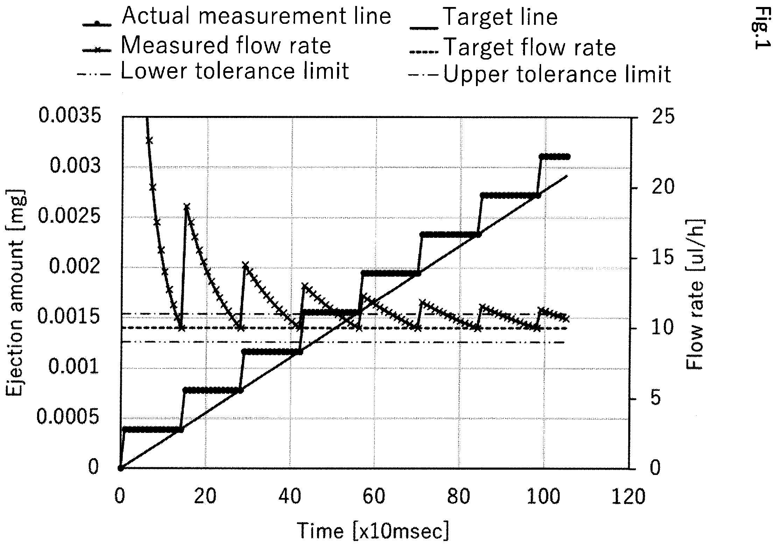

FIG. 1 shows an example of a graph showing a relationship between the measured flow rate of the fluid and the actual integral ejection amount of the fluid based thereon (which can be calculated as an integral value of the measured flow rate) and the time, in the case in which the above-described control is performed. FIG. 1 shows a graph of the measured flow rate of the fluid and a graph of the actual integral ejection amount of the fluid based thereon (hereinafter, referred to as an "actual measurement line"), and also shows a line indicating the integral ejection amount of the fluid that is to be ejected according to the target flow rate (hereinafter, referred to as a "target line"). Furthermore, the target flow rate is indicated by the dotted line, and lines indicating the upper and lower flow rate tolerance limits based on the target flow rate are respectively indicated by a dash-dotted line and a dash-double-dotted line.

As is seen from FIG. 1, in the case in which the above-described intermittent operation is performed, if the actual measurement line becomes even slightly lower than the target line, the pump is driven, and thus the actual measurement line becomes higher than the target line. Then, the pump is turned off, after which, if the actual measurement line again becomes even slightly lower than the target line, the pump is again driven, and the same operation is repeated.

Accordingly, in the above-described control, as shown in FIG. 1, the target line is not at the center of the actual measurement line, and the actual measurement line is biased upward relative to the target line. Furthermore, the measured flow rate may be frequently higher than the upper tolerance limit. As a result, in the above-described control, the trumpet curve deteriorates. Note that the trumpet curve is one of evaluation items regarding the pump performance as defined in JIS (Japanese Industrial Standards) T 0601-2-24:2018, and is obtained by plotting a maximum value of errors on the upper side and a minimum value of errors on the lower side of average measured flow rates of a plurality of measurement time periods (which are respectively referred to as a "maximum measurement error (Ep(max))" and a "minimum measurement error (Ep(min))") relative to the average measured flow rate over the entire measurement section, as well as a difference between the target flow rate and the average measured flow rate over the entire measurement section. Thus, the longer the measurement time periods, the more averaged the measured flow rate, and thus error values are smaller and a graph thereof has a similar shape to that of an instrument called trumpet. Accordingly, the trumpet curve is a curve indicating the level of precision of an ejection amount of a pump with respect to a target value and the variability of a flow rate.

It is an object of the disclosure to provide a pump system with an improved trumpet curve.

A pump system according to a first aspect is a pump system including: a pump configured to intermittently perform an ejecting operation that ejects a fluid; a flow meter configured to measure a flow rate of the fluid ejected from the pump; and a control unit connected to the pump and the flow meter, configured to decide on a length of a stopping section during which the ejecting operation is stopped such that an ejection amount of fluid excessively ejected from the pump is canceled, based on a measured flow rate, which is the flow rate measured by the flow meter, and a target flow rate of the pump set in advance, and configured to control the pump so as to stop the ejecting operation according to the length of the stopping section.

A pump system according to a second aspect is the pump system according to the first aspect, wherein the control unit determines whether or not to stop the ejecting operation, by comparing an ejection amount of fluid ejected from the pump based on the measured flow rate and an ejection amount of fluid that is to be ejected from the pump based on the target flow rate, and decides on the length of the stopping section in a case of stopping the ejecting operation.

A pump system according to a third aspect is the pump system according to the second aspect, wherein it is determined to stop the ejecting operation in a case in which the ejection amount based on the measured flow rate is larger than the ejection amount based on the target flow rate.

According to the above-described aspects, a length of a stopping section during which the ejecting operation of a pump is stopped is decided on such that an ejection amount of fluid excessively ejected from the pump is canceled, based on a measured flow rate measured by a flow meter and a target flow rate of the pump set in advance. Then, the pump is controlled such that the ejecting operation of the pump is stopped according to the decided length of the stopping section. Accordingly, the target line of the flow rate becomes closer to the center of the actual measurement line, and the actual measurement line is prevented from being excessively biased upward relative to the target line. As a result, the trumpet curve of the pump system is improved.

Further embodiments of the invention are described in the following description of the figures and/or the dependent claims attached to this specification. The invention will be explained in the following in detail by means of embodiments and with reference to the drawing in which is shown:

FIG. 1 is an example of a graph showing a relationship between the integral ejection amount of a fluid and the time according to a related technique.
FIG. 2 is a configuration diagram of a pump system according to an embodiment of the disclosure.
FIG. 3A is a side cross-sectional view of the pump during suction.
FIG. 3B is a side cross-sectional view of the pump during ejection.
FIG. 4 is a flowchart showing the flow of feedback control that controls an ejecting operation of the pump based on a measured value of a flow meter.
FIG. 5 is an example of a graph showing a relationship between the integral ejection amount of the fluid and the time according to the embodiment of the disclosure.

In the following the same reference numerals will be used for parts having the same or equivalent function. Any statements made having regard to the direction of a component are made relative to the position shown in the drawing and can naturally vary in the actual position of application.

Hereinafter, a pump system according to an embodiment of the disclosure will be described with reference to the drawings.

1. Configuration of Pump System

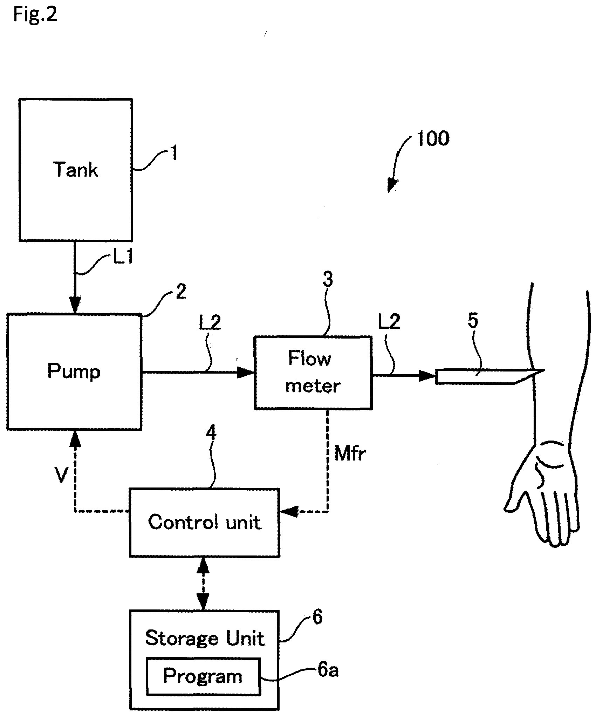

FIG. 2 shows a configuration diagram of a pump system 100 according to this embodiment. As shown in FIG. 2, the pump system 100 includes a tank 1 configured to store a fluid, and a pump 2 arranged downstream of the tank 1 and configured to suck and eject the fluid in the tank 1. In this description, the upstream and the downstream are defined according to the flow of a fluid. The pump 2 intermittently performs an ejecting operation that ejects the fluid.

Furthermore, the pump system 100 further includes a flow meter 3 configured to measure the flow rate of the fluid ejected from the pump 2, and a control unit 4 connected to the pump 2 and the flow meter 3. The control unit 4 controls the fluid transporting operation that is performed by the pump system 100. At this time, the control unit 4 performs feedback control that controls the ejecting operation of the pump 2, based on a measured value Mfr of the flow rate measured by the flow meter 3.

The pump 2 of this embodiment is, but is not limited to, a small or ultra-small pump that enables a fluid to flow at a very low flow rate using a MEMS (micro electro mechanical systems) technique. Quantitatively, the pump 2 may be a small pump whose target flow rate is set to 600 $\mu$l/h or less. However, the pump 2 may also be a smaller pump whose target flow rate is set to 400 $\mu$l/h or less, 200 $\mu$l/h or less, 100 $\mu$l/h or less, 80 $\mu$l/h or less, 60 $\mu$l/h or less, 40 $\mu$l/h or less, or 20 $\mu$l/h or less.

Furthermore, the pump system 100 of this embodiment is, but is not limited to, a system configured to transport a medical fluid such as insulin. Thus, in this embodiment, a needle 5 is connected downstream of the pump 2, and the medical fluid in the tank 1 is given to a patient by the needle 5 being inserted into a patient's arm.

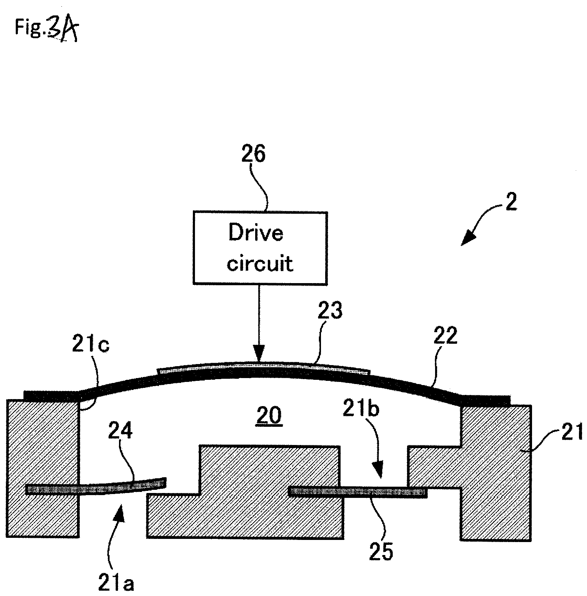

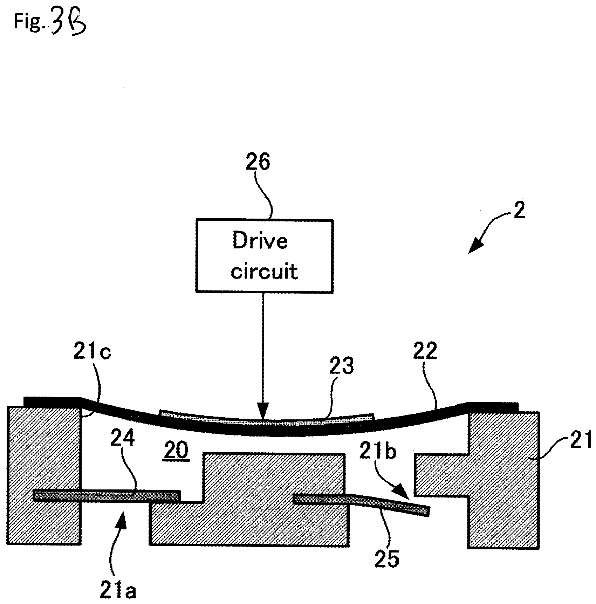

Furthermore, the pump 2 of this embodiment is, but is not limited to, a piezo pump. FIGS. 3A and 3B are side cross-sectional views of the pump 2 illustrating an operating principle of the pump 2. As shown in FIGS. 3A and 3B, the pump 2 includes a casing 21, a diaphragm 22, and a piezo element 23. A pump chamber 20 is arranged inside the casing 21, and a suction port 21a and an ejection port 21b are connected to the pump chamber 20 in the casing. A suction valve 24 configured to open and close the suction port 21a is attached to the port, and an ejection valve 25 configured to open and close the ejection port 21b is attached to the port. Furthermore, an opening 21c is arranged passing through the casing 21, and the diaphragm 22 is attached to the casing 21 so as to close the opening 21c.

The piezo element 23 is attached to the diaphragm 22. The pump 2 further includes a drive circuit 26 configured to drive the piezo element 23. The drive circuit 26 vibrates the piezo element 23, thereby vibrating the diaphragm 22 up and down. The piezo element 23 is constituted by a thin film layer made of a piezoelectric material, and a pair of electrodes respectively arranged on a pair of end faces of the thin film layer, and is vibrated through the application of voltage from the drive circuit 26 to a portion between the electrodes. The drive circuit 26 is connected to the control unit 4, and the control unit 4 controls an operation of the drive circuit 26, thereby controlling vibration of the piezo element 23, and eventually controlling the ejecting operation of the pump 2. A time to drive (turn on) and stop (turn off) vibration of the piezo element 23, that is, the ejecting operation of the pump 2 is controlled by the control unit 4.

When the piezo element 23 is vibrated to deform the diaphragm 22 so as to increase the volume of the pump chamber 20 as shown in FIG. 3A, the pressure inside the pump chamber 20 decreases, and thus the suction valve 24 is opened by being pulled into the pump chamber 20. Accordingly, the fluid in the tank 1 is sucked out via the suction port 21a into the pump chamber 20. The suction port 21a is connected to a first flow path L1, and is further connected via the first flow path L1 to the tank 1.

On the other hand, when the piezo element 23 is vibrated to deform the diaphragm 22 so as to reduce the volume of the pump chamber 20 as shown in FIG. 3B, the pressure inside the pump chamber 20 increases, and thus the ejection valve 25 is opened by being pushed to the outside of the pump chamber 20. Furthermore, at this time, the suction valve 24 is closed by being pushed so as to close the suction port 21a. Accordingly, a fluid is ejected from the pump chamber 20 via the ejection port 21b. The ejection port 21b is connected to a second flow path L2, and the fluid ejected from the pump chamber 20 flows to the downstream side via the second flow path L2 and ultimately reaches the needle 5. Note that, when the piezo element 23 is next vibrated as shown in FIG. 3A, the suction valve 24 is opened, and the ejection valve 25 is closed by being pulled so as to close the ejection port 21b.

The flow meter 3 of this embodiment is, but is not limited to, a flow meter using a MEMS technique. The flow meter 3 is arranged on the second flow path L2, and measures the flow rate of the fluid that is sent out from the pump 2 and flows through the second flow path L2. Furthermore, the flow meter 3 of this embodiment is, but is not limited to, a thermal flow meter.

The control unit 4 is a microcomputer, and is constituted by a CPU, a ROM, a RAM, and the like. The pump system 100 further includes a non-volatile storage unit 6 connected to the control unit 4. The control unit 4 reads and executes a program 6a stored in the storage unit 6, thereby performing the above-described operations. Note that part or the whole of the program 6a may be stored in a ROM constituting the control unit 4.

A target flow rate Tfr of the pump 2 can be set for the pump system 100. The set target flow rate Tfr is stored in the storage unit 6, and is referred to by the control unit 4 as appropriate. There is no particular limitation on the method for setting the target flow rate Tfr of the pump 2, and, for example, it is also possible that an input device such as a button or a switch is mounted in the pump system 100, and a user can input the target flow rate Tfr by operating the device. Alternatively, it is also possible that the control unit 4 is connectable to an external computer, and the target flow rate Tfr input by the user to the computer is transmitted from the computer to the control unit 4 and stored in the storage unit 6. It is assumed that examples of the computer typically include a smartphone, a tablet computer, a desktop computer, and a laptop computer. There is no particular limitation on the form of communicative connection between the computer and the control unit 4, but it may be a wired connection using a cable or wireless connection according to a wireless communication standard such as Bluetooth (registered trademark).

2. Operation of Pump System

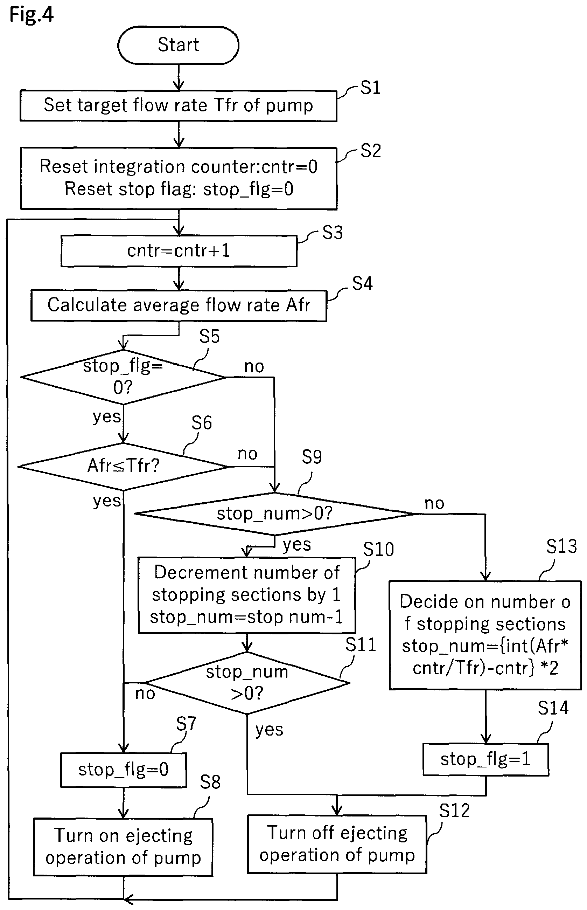

Next, a fluid transporting operation that is performed by the pump system 100 will be described in detail. FIG. 4 is a flowchart showing the flow of feedback control that controls the ejecting operation of the pump 2 based on the measured value Mfr of the flow meter 3, the feedback control being performed during the transporting operation.

First, when driving the pump system 100, the user sets the target flow rate Tfr of the pump 2, for example, by operating an input device mounted in the pump system 100 or an external computer connected to the pump system 100, and stores the target flow rate in the storage unit 6. Furthermore, the user inputs a drive command to drive the pump system 100 to the control unit 4, for example, by operating the input device or the external computer.

Upon receiving input of the above-mentioned drive command, the control unit 4 reads the target flow rate Tfr of the pump 2 from the storage unit 6 (step SI). Furthermore, the control unit 4 resets an integration counter (hereinafter, referred to as a "cntr" as appropriate) to 0, and resets a stop flag (hereinafter, referred to as a "stop_flg" as appropriate) to 0 (step S2).

The integration counter is a counter that measures the length of driving time after the pump system 100 is driven in response to input of the above-mentioned drive command, and is a counter that measures the number of unit sections passed. As will be described later, the control unit 4 decides on whether the ejecting operation of the pump 2 is to be driven (turned on) or stopped (turned off) for each unit section. Note that the ejecting operation of the pump 2 may be continuously driven throughout a plurality of successive unit sections, and, in a similar manner, the ejecting operation of the pump 2 may be continuously stopped throughout a plurality of successive unit sections. In either case, the pump 2 intermittently performs the ejecting operation, and repeatedly drives (turns on) and stops (turns off) the ejecting operation during driving of the pump system 100. The stop flag is a flag for giving an instruction to stop the ejecting operation of the pump 2 in a next unit section following the current unit section, where the flag is set to 1 in the case of stopping the ejecting operation and to 0 in the case of driving the ejecting operation.

After step S2, the control unit 4 increments the value of the integration counter by 1 (step S3). Note that steps S3 to S14 in FIG. 4 are loop processing that is repeatedly performed each time a unit section passes.

Furthermore, when the pump system 100 is driven, the control unit 4 drives the flow meter 3 in each unit section, and causes the flow meter 3 to continuously measure the flow rate of the fluid ejected from the pump 2. The measured value Mfr of the flow rate measured by the flow meter 3 is sequentially transmitted from the flow meter 3 to the control unit 4. After step S3, the control unit 4 calculates an average flow rate Afr of the fluid ejected from the pump 2 from when the pump system 100 is driven to the current point in time, based on the measured value Mfr received from the flow meter 3 (step S4). The average flow rate Afr can be calculated by averaging the measured value Mfr acquired from when the pump system 100 is driven to the current point in time.

After step S4, the control unit 4 refers to the value of the stop flag, and advances the procedure to step S6 if the value is 0 or to step S9 if the value is 1 (step S5). In step S6, the control unit 4 compares the average flow rate Afr calculated in step S4 and the target flow rate Tfr read in step S1, and determines whether or not to stop the ejecting operation of the pump 2. More specifically, if the average flow rate Afr is not greater than the target flow rate Tfr, the control unit 4 determines to drive the ejecting operation in a next unit section following the current unit section (step S6), resets the stop flag to 0 (step S7), and drives the ejecting operation of the pump 2 using the drive circuit 26 (step S8).

On the other hand, if the value of the stop flag is 1 in step S5 or if the average flow rate Afr is greater than the target flow rate Tfr in step S6, the ejecting operation of the pump 2 has to be stopped in one next unit section following the current unit section or in one next unit and a plurality of successive unit sections thereafter, and thus the control unit 4 advances the procedure to step S9. In step S9, the control unit 4 acknowledges the number of unit sections during which the ejecting operation of the pump 2 is stopped (hereinafter, referred to as a "number of stopping sections" and indicated as "stop_num" as appropriate). More specifically, the control unit 4 determines whether or not the number of stopping sections has to be calculated, based on the value of the number of stopping sections.

The procedure advances to step S13 if the number of stopping sections is 0 in step S9, or to step S10 if the number of stopping sections is 1 or more. In step S10, the value of the number of stopping sections is decremented by 1, after which, if the value of the number of stopping sections is still 1 or more (step S11), the ejecting operation of the pump 2 is stopped using the drive circuit 26 (step S12). On the other hand, if the number of stopping sections is 0 in step S11, the stop flag is reset to 0 (step S7), and the ejecting operation of the pump 2 is started using the drive circuit 26 (step S8).

Incidentally, it is possible to convert the above-described average flow rate Afr into the ejection amount of fluid ejected from the pump 2 from when the pump system 100 is driven to the current point in time (hereinafter, referred to as an "actual integral ejection amount"), by considering the length of driving time of the pump system 100 by referring to the value of the integration counter, for example. In a similar manner, it is also possible to convert the above-described target flow rate Tfr into the ejection amount of fluid that is to be ejected from the pump 2 from when the pump system 100 is driven to the current point in time according to the target flow rate Tfr (hereinafter, referred to as a "target integral ejection amount"). Accordingly, it can be said that comparing the average flow rate Afr and the target flow rate Tfr in step S6 is the same as comparing the actual integral ejection amount specified based on the measured value Mfr and the target integral ejection amount specified based on the target flow rate Tfr.

As described above, step S9, in which the number of stopping sections is acknowledged, is performed in the case in which it is determined to stop the ejecting operation in a next unit section following the current unit section. If the number of stopping sections is 0 in step S9, the control unit 4 decides on the length of the stopping section of the ejecting operation (step S13). In this embodiment, the length of the stopping section is calculated as the number of stopping sections. More specifically, the number of stopping sections is calculated according to Equation 1 below. Note that, in Equation 1, int() is a function that returns the largest integer that is not larger than the numerical value in ().

$$\text{stop\_num} = \{\text{int}(Afr*cntr/Tfr) - cntr\}*2$$

$$(\text{Equation } 1)$$

As a description of the meaning of Equation 1, first, the target flow rate Tfr is achieved when Equation 2 below holds up.

$$Tfr = (Afr*cntr + 0*stop\_num')/(stop\_num' + cntr)$$

$$(\text{Equation } 2)$$

Note that "stop_num' " is a necessary number of unit sections during which the pump 2 is stopped in order to make the average flow rate Afr match the target flow rate Tfr, for the average flow rate Afr that is greater than the target flow rate Tfr.

Then, stop_num' = Afr*cntr/Tfr-cntr is obtained by transforming Equation 2, and stop num' = int(Afr*cntr/Tfr)-cntr is obtained by expressing the first term on the right hand side as an integer. At this time, the average flow rate Afr is biased upward relative to the target flow rate Tfr, and, in order to make the average flow rate Afr uniform relative to the target flow rate Tfr on the upper and lower sides, it is desirable to stop the pump 2 during the number of unit sections that is twice the number obtained with stop num'. Accordingly, Equation 1 is obtained.

As described above, in step S13, the number of stopping sections indicating the length of the stopping section during which the ejecting operation of the pump 2 is stopped is decided based on the average flow rate Afr based on the measured value Mfr of the flow meter 3, the target flow rate Tfr set in advance, and the integration counter cntr indicating the driving time of the pump system 100. At this time, as is clear from Equation 1, the number of stopping sections is decided on so as to cancel the ejection amount of fluid excessively ejected from the pump 2 from when the pump system 100 is driven to the current point in time. The excessive ejection amount means an excessive amount relative to the target integral ejection amount, contained in the actual integral ejection amount.

After step S13, the control unit 4 sets the stop flag to 1 (step S14), and then stops the ejecting operation of the pump 2 using the drive circuit 26 (step S12). After step S8 or S12, the procedure returns to step S3, and similar steps are repeated.

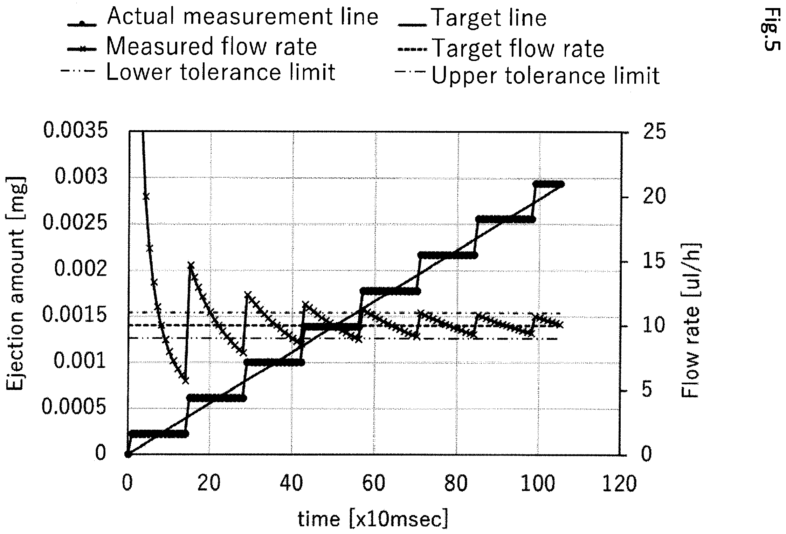

FIG. 5 is an example of a graph showing a relationship between the actually measured flow rate of the fluid and the actual integral ejection amount of the fluid based thereon, and the time, in the case in which the feedback control according to this embodiment is performed. As in FIG. 1, FIG. 5 shows an actual measurement line indicating the actually measured flow rate of the fluid and the integral ejection amount, and also shows a target line indicating the target integral ejection amount. Furthermore, the target flow rate is indicated by the dotted line, and lines indicating the upper and lower flow rate tolerance limits based on the target flow rate are respectively indicated by a dash-dotted line and a dash-double-dotted line.

As is seen from the comparison between FIGS. 1 and 5, in this embodiment, the actual measurement line is prevented from being excessively biased upward relative to the target line contrary to the case of FIG. 1. Furthermore, the measured flow rate is, over time, unlikely to be out of the region defined by lines indicating the upper and lower flow rate tolerance limits. That is to say, in the example in FIG. 1, the pump 2 is immediately driven when the actual measurement line becomes even slightly lower than the target line, and thus the actual measurement line is biased upward relative to the target line. On the other hand, in this embodiment, the pump 2 is not necessarily immediately driven when the actual measurement line becomes lower than the target line. That is to say, in this embodiment, after the stopped state of the pump 2 is maintained for a while as necessary until the ejection amount of fluid excessively ejected from the pump 2 is canceled, the pump 2 is driven again. As a result, the target line becomes closer to the center of the actual measurement line, and thus the trumpet curve of the pump system 100 is improved. Note that the trumpet curve is one of evaluation items regarding the pump performance, and is a curve indicating the level of precision of an ejection amount of a pump with respect to a target value and the variability of a flow rate. In this embodiment, the target flow rate Tfr is precisely achieved based on the improved trumpet curve. More specifically, the values of the maximum measurement error (Ep(max)) and the minimum measurement error (Ep(min)) obtained from the trumpet curve can be effectively made uniform.

While the above-described feedback control is performed, the fluid intermittently ejected from the pump 2 flows through the second flow path L2 and is sent into a patient by the needle 5 being inserted into a patient's body. In this embodiment, it is possible to precisely give a desired amount of fluid to the patient's body, based on the improved trumpet curve.

3. Modified Examples

In the description above, an embodiment of the disclosure has been described, but the disclosure is not limited to the foregoing embodiment, and can encompass various modifications without departing from the gist thereof. For example, the following modifications are possible.

3-1

In the foregoing embodiment, a small or ultra-small pump using a MEMS technique was given as an example of the pump 2, but there is no limitation to this. Furthermore, in the foregoing embodiment, a piezo pump was given as an example of the pump 2, but there is no limitation to this. Various types of pumps may be used as the pump 2.

3-2

The fluid that is transported by the pump system 100 is not limited to a liquid, and may also be a gas.

LIST OF REFERENCE NUMERALS

100    Pump system
1      Tank
2      Pump
3      Flow meter
4      Control unit
5      Needle
6      Storage unit


**Claims**

1. A pump system (100) comprising:

   a pump (2) configured to intermittently perform an ejecting operation that ejects a fluid;
   a flow meter (3) configured to measure a flow rate of the fluid ejected from the pump; and
   a control unit (4) connected to the pump (2) and the flow meter (3), configured to decide on a length of a stopping section during which the ejecting operation is stopped such that an ejection amount of fluid excessively ejected from the pump (2) is canceled, based on a measured flow rate, which is the flow rate measured by the flow meter (3), and a target flow rate of the pump (2) set in advance, and configured to control the pump (2) so as to stop the ejecting operation according to the length of the stopping section.

2. The pump system (100) according to claim 1, wherein the control unit (4) determines whether or not to stop the ejecting operation, by comparing an ejection amount of fluid ejected from the pump (2) based on the measured flow rate and an ejection amount of fluid that is to be ejected from the pump (2) based on the target flow rate, and decides on the length of the stopping section in a case of stopping the ejecting operation.

3. The pump system (100) according to claim 2, wherein it is determined to stop the ejecting operation in a case in which the ejection amount based on the measured flow rate is larger than the ejection amount based on the target flow rate.

4. The pump system (100) according to one of claims 1 to 3, wherein the voltage is a control parameter for increasing or decreasing an ejection amount of the pump (2); and/or
   wherein the flow rate of the pump (2) is controlled by increasing or decreasing an ejection amount of fluid.

5. The pump system (100) according to one of claims 1 to 4, wherein the pump (2) includes a casing (21), a diaphragm (22), and a piezo element (23).

6. The pump system (100) according to claim 5, wherein a pump chamber (20) is arranged inside the casing (21), and a suction port (21a) and an ejection port (21b) are connected to the pump chamber (20) in the casing (21).

7. The pump system (100) according to claims 5 and 6, wherein a suction valve (24) configured to open and close the suction port (21a) is attached to the suction port (21a), and an ejection valve (25) configured to open and close the ejection port (21b) is attached to the ejection port (21b).

8. The pump system (100) according to one of claims 5 to 7, wherein an opening (21c) is arranged passing through the casing (21), and wherein the diaphragm (22) is attached to the casing (21) so as to close the opening (21c).

9. The pump system (100) according to one of claims 5 to 8, wherein the piezo element (23) is attached to the diaphragm (22).

10. The pump system (100) according to one of claims 5 to 9, wherein the pump (2) further includes a drive circuit (26) configured to drive the piezo element (23).

11. The pump system (100) according to claim 10, wherein the drive circuit (26) is configured to vibrate the piezo element (23).

12. The pump system (100) according to one of claims 5 to 11, wherein the piezo element (23) comprises a thin film layer made of a piezoelectric material, and a pair of electrodes respectively arranged on a pair of end faces of the thin film layer, and wherein a voltage V can be applied from the drive circuit (26) to a portion between the electrodes.

13. The pump system (100) according to one of claims 9 to 12, wherein the drive circuit (26) is connected to the control unit (4), and the voltage (V) that is applied via the drive circuit (26) to the piezo element (23) is controlled by the control unit (4).

14. The pump system (100) according to one of claims 1 to 13, wherein the control unit (4) is connectable to an external computer, and the target flow rate input by a user to the computer is transmitted from the computer to the control unit (4) and is stored in a storage unit (6).

15. The pump system (100) according to one of claims 1 to 14, wherein the control unit (4) is a microcomputer, and comprises at least one of the following: a CPU, a ROM, a RAM, and the like.

Fig.1

Fig.2

Fig.3A

Fig. 3B

Fig.4

Fig.5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011160868 A